# EUROPEAN PATENT SPECIFICATION

(11) **EP 4 025 099 B1**
(45) Date of publication and mention of the grant of the patent: **23.07.2025**
(21) Application number: 20761844.8
(22) Date of filing: 28.08.2020
(51) Int. Cl.: A46B 15/00, G16H 20/30, G16H 50/30, A61C 17/22

(54) **PROPORTIONAL DIVISION OF A TOTAL OPERATION TIME OF A DENTAL CARE PROCEDURE**
PROPORTIONALE AUFTEILUNG EINER GESAMTBETRIEBSZEIT EINES ZAHNPFLEGEVERFAHRENS
DIVISION PROPORTIONNELLE D'UN TEMPS DE FONCTIONNEMENT TOTAL D'UNE OPÉRATION DE SOINS DENTAIRES

(30) Priority: 05.09.2019 EP 19195493
(43) Date of publication of application: 13.07.2022
(73) Proprietor: Koninklijke Philips N.V., 5656 AG Eindhoven (NL)
(72) Inventor: RMAILE, Amir, Hussein, 5656 AE Eindhoven (NL); NUNES, Dionisio, Massingo, 5656 AE Eindhoven (NL)
(74) Representative: Philips Intellectual Property & Standards
(86) International application number: PCT/EP2020/074039
(87) International publication number: WO 2021/043672

(56) References cited:
- WO-A1-2018/087627
- WO-A1-2018/087627
- KR-A- 20170 075 334
- KR-A- 20170 075 334
- US-A1- 2010 106 336
- US-A1- 2011 010 876
- US-A1- 2011 010 876
- US-A1- 2012 137 454
- US-A1- 2012 137 454
- US-A1- 2018 132 603
- US-A1- 2018 132 603

## Description

### FIELD OF THE INVENTION

In the first place, the invention relates to a system that is configured to be operated in a context of a dental care procedure to be performed in the mouth of a user for a total operation time, the dental care procedure including dental care actions to be performed at respective areas in the user's mouth by means of a functional unit, and the system comprising an appliance that is configured to operate such a functional unit.

In the second place, the invention relates to a controller that is configured to execute an algorithm for dividing a total operation time of a dental care procedure to be performed in a person's mouth in effective time durations of a dental care action per area in the person's mouth.

In the third place, the invention relates to a remote device, comprising a controller as mentioned and being configured to communicate with a system that is configured to be operated in a context of a dental care procedure to be performed in the mouth of a user for a total operation time, the dental care procedure including dental care actions to be performed at respective areas in the user's mouth by means of a functional unit, and the system comprising an appliance that is configured to operate such a functional unit, wherein the controller is configured to control operation of the appliance in accordance with the operation data.

In the fourth place, the invention relates to an appliance that is configured to operate a functional unit for the purpose of performing dental care actions at respective areas in a user's mouth, the appliance comprising the controller as mentioned, wherein the controller is configured to control operation of the appliance in accordance with the operation data.

In the fifth place, the invention relates to a computer program product comprising code to cause a controller, when the code is executed on the controller, to execute an algorithm for dividing a total operation time of a dental care procedure to be performed in a person's mouth in effective time durations of a dental care action per area in the person's mouth.

### BACKGROUND OF THE INVENTION

US 5,544,382 relates to a system as mentioned in the foregoing. In particular, US 5,544,382 discloses a power toothbrush which includes a support member having a brush-head at a distal end thereof and means for driving the brush-head. The power toothbrush further includes a timing means, responsive to the device being turned on, for producing successive indications of intervals of elapsed time, wherein the intervals of elapsed time are related to desired times for brushing portions of the dental region. For instance, a 120-second timer is used, and operation of the toothbrush hence stops after 120 seconds after having been turned on by a user. A total time duration of a brushing procedure of 120 seconds or 2 minutes is generally considered as an optimal time duration involving satisfactory dental cleaning results. In the context of US 5,544,382, it is stated that a most productive practical time/area division was found to be four identical time intervals, hence in effect dividing brushing time between four quadrants of the dental region. Various options for communicating the end of a 30-second interval to a user are addressed in US 5,544,382, including options of producing an audible signal or interrupting the means for driving the brush-head with a "silence" burst.

US 2012/137454, US 2011/010876, KR 2017/0075334, WO 2018/087627, and US 2018/132603 A1 disclose prior art examples.

### SUMMARY OF THE INVENTION

It is an object of the invention to improve the prior art. The invention is defined by the independent claims. The dependent claims define advantageous embodiments.

In the following, the features of the invention will be explained in the context of tooth brushing, which is not to be understood so as to imply that the invention is limited to such a context in any way nor that the following information is intrinsically linked to such a context in any way. The invention is also applicable to the context of flossing, irrigating, bracket cleaning, to mention a few other examples, as well as to combinations of two or even more contexts.

As mentioned in the foregoing, a total time duration of a brushing procedure of 120 seconds or 2 minutes is generally considered as an optimal time duration involving satisfactory dental cleaning results. In view thereof, dentists commonly recommend a 2-minute brushing time to their patients, and many power toothbrushes are programmed to follow a 2-minute brushing regime. The programmed 2-minute regime may be evenly distributed over four quadrants of the dental region, or another number of segments may be chosen, as known from US 5,544,382. The invention involves an insight that such a distribution of the total brushing time does not take into consideration the individual state of each tooth/quadrant/segment, and also that a distinction between teeth which are more susceptible to a dental condition and teeth which are less susceptible to a dental condition is not made. In the known systems, a tooth with a high probability of having pockets or caries is given the same amount of brushing time as a tooth with a low probability of having pockets or caries.

By providing a system comprising an appliance as defined in the foregoing, combined with a data providing arrangement and a controller as defined in the foregoing, the invention offers a possibility of obtaining a far more sophisticated time/area division than the ones hitherto known/suggested in the art. The invention proposes not to have the conventional one-size-fits-all approach when it comes to distributing a total brushing time over the whole mouth, but to take into consideration variations relating to a person or an entire population instead and to make appropriate adjustments on the basis thereof. In particular, it may be so that the distribution of the extent to which dental care is needed at the areas of the respective combinations is based on a distribution of likelihood of disease development at the areas of the respective combinations. Also, it may be so that the distribution of the extent to which dental care is needed at the areas of the respective combinations is based on knowledge of the user's personal information and/or dental information.

A possible way of putting the invention to practice involves ranking all the teeth according to likelihood/probability of developing pockets or caries on the basis of dental/periodontal data extracted from a large population. The brushing time is automatically distributed over all the teeth/quadrants/segments in the mouth based on this ranking, so that the brushing time is distributed over the teeth in a "fair" way, wherein teeth with a high likelihood/probability of developing pockets or caries get the lion's share of the brushing time, while teeth with a low likelihood/probability of developing pockets or caries get a significantly smaller share that may be no more than a required minimum. This may result in a distribution of the brushing time including different effective time durations of a brushing action per tooth/quadrant/segment in the mouth, wherein a number of different effective time durations may be two or (many) more.

In no way is the invention restricted to the practical example of 2 minutes known from the field of tooth brushing when it comes to the total operation time. When the invention is applied in the field as mentioned, it may even turn out to be so that the total operation time can be chosen so as to be smaller and to still have appropriate effective time durations attributed to each of the areas in the user's mouth. After all, there is no scientific evidence that a total brushing time of 2 minutes is optimal.

The invention involves a possibility of developing toothbrushes and other systems for use in a dental care context which are designed to be in compliance with the average needs of a population when it comes to realizing various extents of dental care in various areas in the mouth. Also, the invention involves a possibility of providing systems for use in a dental care context which are adaptable to the needs of a specific user, in which case the systems may be equipped with a user interface and a programmable data providing arrangement and/or controller.

It may be practical if the system is equipped with means for real-time detection of the positions where the dental care actions are performed during a dental care procedure. Contrariwise, in case the positions where the dental care action is performed are automatically set by the controller, which may be the case if the functional unit comprises some kind of mouthpiece that is configured to be placed over a number of teeth during use, for example, the system may do without such position detection means, although such means could still be provided for providing feedback about actual positions and making corrections on the basis of such feedback. In the words of the definition of the system according to the invention, this implies that the controller may be configured to provide the data providing arrangement with input relating to an actual area where a dental care action is performed during operation and to retrieve output relating to an actual effective time duration of the dental care action from the data providing arrangement, and that alternatively or additionally, the controller may comprise an area detector arrangement that is configured to detect an actual area where a dental care action is performed during operation.

Within the framework of the invention, any suitable way of ranking various areas in the mouth may be applied. **In** this respect, it is noted that it may be practical if use is made of care values representing the extent to which dental care is needed at the areas of the respective combinations making up the operation data included in the data providing arrangement. For example, such care values may be directly proportional to the extent to which dental care is needed, in which case a higher care value represents a higher need of dental care, and a lower care value represents a lower need of dental care.

The invention is not limited to a particular type of dental care, and the values of the respective effective time durations, which will hereinafter be referred to as time values, may be determined on the basis of area-dependent needs in respect of one or more types of dental care. For example, the distribution of the time values may be related to a distribution of pocket depth per tooth, wherein the pocket depth may be an average pocket depth that is found in a representative population or an actual pocket depth in a specific user's mouth, and wherein higher time values may be associated with relatively deep pockets and lower time values may be associated with not so deep pockets. **In** general, it may be so that at least a portion of care values representing an extent to which dental care is needed are average values which are applicable to a population of users and/or that at least a portion of such care values are values which are applicable to a specific user.

As mentioned in the foregoing, the system may comprise a user interface that is configured to allow a person to adjust the operation data in the data providing arrangement, so that the way in which the system is operable can be adjusted to specific needs. For example, the system may be configured to enable a situation in which a dental practitioner adapts settings of the system on the basis of patient-specific mouth features such as the absence of one or more teeth, the presence of one or more crowns, the occurrence of periodontitis at one or more positions in the mouth, etc., which does not alter the fact that the system may generally be user-friendly and may be suitable to be adjusted by patients/users themselves as well. The possible user interface may be provided in any suitable form, allowing persons to provide input to the system in any suitable way. **In** this respect, it is noted that use may be made of any appropriate means of communication such as the Internet. The system may comprise a remote device on which an app is installed that is programmed to receive input provided by a person and convey such input to the data providing arrangement and/or the controller.

In respect of the option of settings of the system being adjustable, it is noted that it may be practical for the controller to be configured to execute an algorithm for adjusting the operation data in the data providing arrangement on the basis of input in respect of at least one area in the user's mouth, which algorithm comprises the steps of: i) adjusting the extent to which dental care is needed in respect of the at least one area, ii) determining a new distribution of the extent to which dental care is needed at the areas of the respective combinations, and iii) making a new proportional division of the total operation time on the basis of the new distribution of the extent to which dental care is needed at the areas of the respective combinations. In such a case, it may further be so that the data providing arrangement includes data in the form of respective combinations of an area and a minimum effective time duration, and wherein the algorithm comprises the steps of: i) checking whether the adjusted operation data involve one or more combinations of area and effective time duration in which the effective time duration is an unallowable effective time duration that is below the minimum effective time duration related to the respective area, and ii) increasing any unallowable effective time duration to the minimum effective time duration related to the respective area. This is one way of ensuring that each of the areas will be subjected to the dental care action for a sufficient amount of time, i.e. at least a minimum amount of time. The outcome of following the steps as mentioned leads to an adjusted division of the total operation time which may or may not involve an extension of the total operation time in comparison to the initial adjustment. If an extension of the total operation time is undesirable, yet further adjustments may be made by performing further steps aimed at maintaining an original total operation time, wherein a further new proportional division of the total operation time is calculated while taking into account all minimum effective time durations and probably other factors, i.e. correction factors.

Depending on requirements and expectations in respect of the dental care procedure to be performed, the total operation time may or may not be adjustable in the data providing arrangement. It may be practical to have a default of the total operation time, which default may be 2 minutes as mentioned earlier in respect of toothbrushing, or any other suitable amount of time.

Advantageously, the system according to the invention comprises a communication arrangement that is configured to alert a person to the expiry of an effective time duration at an actual area where a dental care action is performed during operation. Having such a communication arrangement may especially be beneficial in case the functional unit is movable through the mouth by hand, as a person manipulating the functional unit may rely on the information communicated to him/her through the communication arrangement for ensuring that a dental care procedure takes place in a correct manner as envisaged in such a case.

The respective areas of the respective combinations making up the operation data included in the data providing arrangement may relate to respective teeth and/or respective mouth segments including at least two teeth, which does not alter the fact that other options are covered by the invention as well, including the option of the respective areas relating to respective parts of respective teeth.

As mentioned earlier, the system according to the invention may comprise a remote device on which an app is installed that is programmed to receive input provided by a person and convey such input to the data providing arrangement and/or the controller. In case such a remote device is used, indeed, it is also possible that the app is programmed to provide information to a person including information about effective time durations during operation of the system, as an alternative or as an addition. For the sake of completeness, it is noted that practical examples of a remote device include smartphones, tablets and laptops.

The system according to the invention may actually comprise the functional unit that is configured to perform the dental care action included in the dental care procedure. A practical example of such a functional unit is a brush-head.

The concept of the invention, i.e. the concept of making a proportional division of a total operation time on the basis of a distribution of an extent to which dental care is needed at respective areas in the mouth, is very well applicable to a method of designing a system that is configured to be operated in a context of a dental care procedure to be performed in the mouth of a user for a total operation time by subjecting respective areas in the user's mouth to a dental care action. In particular, according to the invention, such a method may comprise a step of determining operation data in the form of respective combinations of an area in the user's mouth and an effective time duration of the dental care action to be performed at the area concerned, wherein an extent to which dental care is needed at the areas is rated, and wherein a proportional division of the total operation time is made on the basis of a distribution of the rated extent to which dental care is needed. The step as mentioned may also be included in a method of determining control settings of an appliance that is configured to operate a functional unit configured to perform dental care actions at respective areas in a user's mouth.

The invention further provides a controller that is configured to execute an algorithm for dividing a total operation time of a dental care procedure to be performed in a person's mouth in effective time durations of a dental care action per area in the person's mouth, which algorithm is designed to process information about a distribution of the extent to which dental care is needed over the respective areas in the person's mouth, and to determine operation data in the form of respective combinations of an area in the person's mouth and an effective time duration of the dental care action to be performed at the area concerned by making a proportional division of the total operation time on the basis of the distribution of the extent to which dental care is needed over the respective areas in the person's mouth. In respect of the step of processing information about a distribution of the extent to which dental care is needed over the respective areas in the person's mouth, it is noted that such step may comprise sub steps of retrieving care values representative of an extent to which dental care is needed at the respective areas in the person's mouth, and determining a distribution of the care values over the respective areas in the person's mouth. The invention also provides a computer program product comprising code to cause a controller, when the code is executed on the controller, to execute the algorithm as mentioned.

The above-described and other aspects of the invention will be apparent from and elucidated with reference to the following detailed description of ways in which a proportional division of a total operation time of a dental care procedure is made when the invention is put to practice.

### BRIEF DESCRIPTION OF THE DRAWINGS

The invention will now be explained in greater detail with reference to the figures, in which equal or similar parts are indicated by the same reference signs, and in which:
Fig. 1 is a block diagram of an embodiment of the system according to the invention;
Fig. 2 is a graph showing an average pocket depth per tooth, extracted from a data set relating to a human population; and
Fig. 3 illustrates a division of the human mouth in six segments.

### DETAILED DESCRIPTION OF EMBODIMENTS

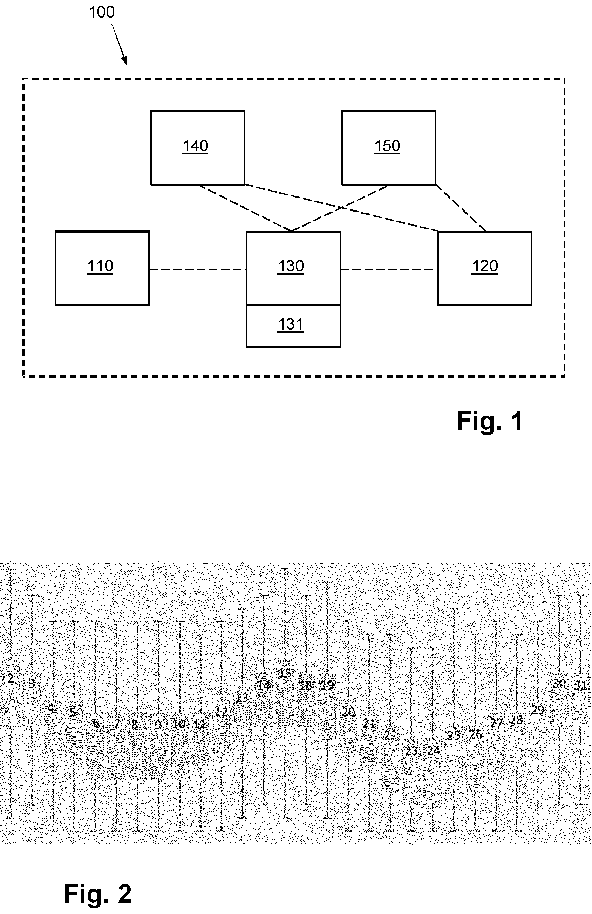

Fig. 1 illustrates the set-up of an embodiment of the system 100 according to the invention by depicting various components of the system 100 as boxes. In the following, the various components are named and further defined.

In general, the system 100 is configured to be operated in a context of a dental care procedure to be performed in the mouth of a user for a total operation time. The system 100 may be suitable to be used on a regular basis, which may especially the case when the dental care procedure involves brushing teeth, spraying liquid towards teeth and interdental spaces, etc.

In the first place, the system 100 comprises an appliance 110 that is configured to operate a functional unit for subjecting respective areas in the user's mouth to a dental care action. For example, the functional unit may include a movable brush-head and/or a nozzle for emitting liquid, and the appliance 110 may be a handle that is configured to support and drive the functional unit. In general, it may be practical for the functional unit to be removable from the appliance 110, although this is not essential within the framework of the invention. Further, the functional unit may be of such a design that an effective positioning of the functional unit is a positioning in which the functional unit is at least partially inserted in the user's mouth.

In the second place, the system 100 comprises a data providing arrangement 120 that is configured to include operation data in the form of respective combinations of an area in the user's mouth and an effective time duration of the dental care action to be performed at the area concerned, wherein the effective time durations of the respective combinations add up to the total operation time of a dental care procedure and have respective values according to a proportional division of the total operation time on the basis of a distribution of the extent to which dental care is needed at the areas of the respective combinations. Ways in which the proportional division of the total operation time are made are explained later. By having the data providing arrangement 120 that is configured to include the operation data as defined, the system 100 offers the possibility of closely/fully complying with any variation in need for dental care throughout the mouth. In practice, the data providing arrangement 120 may comprise any suitable type of database system.

In the third place, the system 100 comprises a controller 130 that is configured to communicate with the data providing arrangement 120 and to control operation of the appliance 110 in accordance with the operation data. In the shown example, the controller 130 comprises an area detector arrangement 131 that is configured to detect an actual area where a dental care action is performed during operation. Information about an actual area where a dental care action is performed is useful input for determining an actual effective time duration of the dental care action. Besides being able to follow algorithms for realizing operation of the appliance 110 in accordance with the operation data, the controller 130 may be configured to follow more operation-related algorithms such as turning the appliance 110 on and off, respectively, when user input to that end is received, controlling movement of specific parts of the functional unit, etc.

In the framework of the invention, the presence of an area detector arrangement in the system 100 is optional. The components listed in the following are also not essential to the very gist of the invention, yet may involve advantageous functionalities and are therefore presented as being included in the present embodiment of the system 100.

In the fourth place, the system 100 comprises a user interface 140 that is configured to allow a person to adjust the operation data in the data providing arrangement 120. A process of adjusting the operation data in the data providing arrangement 120 may involve execution of one or more algorithms programmed in the controller 130.

In the fifth place, the system 100 comprises a communication arrangement 150 that is configured to alert a person to the expiry of an effective time duration at an actual area where a dental care action is performed during operation.

At least one of the user interface 140 and the communication arrangement 150 may be realized by providing a remote device on which an app is installed that is programmed to receive input provided by a person and convey such input to the data providing arrangement 120 and/or the controller 130, and/or to provide information to a person including information about effective time durations during operation of the system 100.

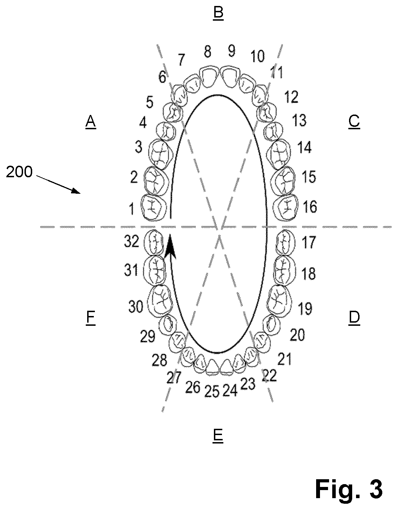

An example of data as may be at the basis of the proportional division of the total operation time is provided by means of Fig. 2. In particular, in Fig. 2, an average pocket depth per tooth is plotted, extracted from a data set of thousands of people, i.e. a data set representative of a human population. In the context of the population as mentioned, Fig. 2 shows a distribution of pocket depth per every tooth in the mouth, wherein each box shown in the figure represents an average pocket depth of a certain tooth that is identified by a number. The numbers identifying the various teeth can also be found in Fig. 3. In the shown example, teeth identified by the numbers 2, 3, 14, 15, 18, 30 and 31 involve a relatively large average pocket depth, while teeth identified by the numbers 23 and 24 involve a relatively small average pocket depth.

The following example table shows how the graph of Fig. 2 can be interpreted to rank the teeth according to a likelihood of having deeper pockets. Further, the table shows how a total operation time of 2 minutes can be divided between the various teeth on the basis of ratio factors attributed to the teeth on the basis of the associated pocket depths, wherein a ratio factor of teeth involving pocket depths in a smallest range is set to be 1, and wherein ratio factors of teeth involving pocket depths in higher ranges are set as an appropriate multiplication of 1. Hence, the present system, a higher ratio factor represents a higher need of dental care, and a lower ratio factor represents a lower need of dental care.

| Tooth number | Ratio factor | Effective time duration of dental care action at tooth (seconds) |
|---|---|---|
| 15 | 3,5 | 7 |
| 2 | 3,5 | 7 |
| 3 | 3,5 | 7 |
| 14 | 3,5 | 7 |
| 18 | 3,5 | 7 |
| 30 | 3,5 | 7 |
| 31 | 3,5 | 7 |
| 19 | 3 | 6 |
| 13 | 2,5 | 5 |
| 12 | 2,5 | 5 |
| 4 | 2,5 | 5 |
| 5 | 2,5 | 5 |
| 29 | 2,5 | 5 |
| 20 | 2,5 | 5 |
| 28 | 1,5 | 3 |
| 21 | 1,5 | 3 |
| 27 | 1,5 | 3 |
| 11 | 1,5 | 3 |
| 6 | 1,5 | 3 |
| 7 | 1,5 | 3 |
| 8 | 1,5 | 3 |
| 9 | 1 | 2 |
| 10 | 1 | 2 |
| 22 | 1 | 2 |
| 26 | 1 | 2 |
| 25 | 1 | 2 |
| 23 | 1 | 2 |
| 24 | 1 | 2 |

In the context of the example of a dental care procedure to be performed on 28 teeth in a total operation time of 120 seconds, assuming that appropriate fractions of a total operation time are determined on the basis of a distribution of the need for dental care, as may be derived from a distribution of pocket depth as demonstrated in the foregoing and/or from a distribution of one or more other relevant features, numerous other proportional divisions of the total operation time are possible. In another arbitrary example, the distribution of at least one relevant feature over the 28 teeth may be such that an effective time duration of 10 seconds is attributed to three of the teeth, an effective time duration of 6 seconds is attributed to six of the teeth, an effective time duration of 5 seconds is attributed to four of the teeth, an effective time duration of 4 seconds is attributed to one of the teeth, an effective time duration of 3 seconds is attributed to two of the teeth, and an effective time duration of 2 seconds is attributed to twelve of the teeth. In such a case, the total operation time is divided in six different effective time durations, namely 10 seconds, 6 seconds, 5 seconds, 4 seconds, 3 seconds and 2 seconds, whereas in the example to which the above table relates, the number of different effective time durations is five.

If one or more of the longest effective time durations which are found as a result of making an appropriate proportional division of the total operation time appear to be longer than actually necessary, such a situation offers a possibility of choosing a reduction of the total operation time without compromising dental care effects as envisaged. On the other hand, if one or more of the shortest effective time durations which are found as a result of making an appropriate proportional division of the total operation time appear to be shorter than a required minimum time duration, this may be compensated for by prolonging the total operation time or making a proportional division in which minimum time durations are taken into account. Every possibility as desired can be realized on the basis of configuring the controller 130 so as to follow appropriate algorithms in a process of determining operation data.

In the example of the effective time durations being determined per tooth, in order to fully comply with the need of dental care per tooth, it is most accurate if those effective time durations are taken into account during operation of the system 100. However, it may be more practical not to have a division per tooth but per segment. For example, as illustrated in Fig. 3, the human mouth 200 (as a total of both the upper jaw and the lower jaw) may be divided in an appropriate number of segments including more than one teeth, wherein an optimal time to be spent per segment for performing a dental care action in the segment is determined by making an addition of the effective time durations of the teeth in those segments. With reference to the details of the example that is at the basis of the above table, assuming that a division of the mouth 200 in six segments A, B, C, D, E, F is made, it may be appropriate to attribute 24 seconds of the total operation time to segment A, 16 seconds of the total operation time to segment B, 24 seconds of the total operation time to segment C, 21 seconds of the total operation time to segment D, 13 seconds of the total operation time to segment E, and 22 seconds of the total operation time to segment F.

In the following, ways in which the system 100 according to the invention can be used will be described, whereby the functionalities of the various components of the system 100 are further elucidated.

It follows from the foregoing that the system 100 can be used for the purpose of providing dental care in a way that is determined by taking into account mouth features of a population. In the present embodiment of the system 100, it is possible to make further adaptations as deemed necessary for complying with user-specific dental care needs. In the example of a dental practitioner and a patient, it may be so that the dental practitioner establishes a treatment plan for the patient and conveys dental information about the patient to the data providing arrangement 120 and/or the controller 130 through the user interface 140, particularly dental information related to an extent of dental care needed at various areas in the mouth 200 (e.g., various teeth). As explained earlier, in practice, this may be realized by the dental practitioner using a remote device running an app. On the basis of the input provided through the user interface 140, appropriate operation data are determined by making a proportional division of the total operation time on the basis of a distribution of the extent to which dental care is needed at respective areas in the mouth 200 following from the input. As an outcome of the setting process, a personalized distribution of a recommended total operation time over all the teeth in the mouth 200 is obtained. It may also be possible to provide input relating to the total operation time, for increasing or decreasing the total operation time. Further, the system 100 may provide a recommendation in respect of the total operation time through the communication arrangement 150, which may or may not be accepted.

Examples of patient-specific data include the following: i) one or more teeth may be missing, so that an effective time duration of zero can be attributed to those teeth, ii) features such as one or more teeth with fillings, crowns, implants, partial dentures, localized braces on a dental arch etc. may be present in the mouth 200, and the extent to which dental care is needed for such features can be taken into account in the process of making the proportional division of the total operation time, iii) one or more areas of the mouth 200 may be affected by periodontitis, so that a relatively high extent to which dental care is needed is applicable to those areas, and iv) dental pockets which are deeper than the average dental pockets may be present at one or more teeth, which also involves an increase of the extent to which dental care is needed in respect of those teeth and thus prolonged effective time durations of the dental care actions to be performed at those teeth.

In the present embodiment of the system 100, the functional unit is to be moved through the mouth 200 by a person holding at least part of the appliance 110. Operation of the system 100 involves the following aspects: i) the person manipulating the appliance 110 puts the functional unit at an appropriate position with respect to an area to be subjected to a dental care action, ii) the area detector arrangement 131 detects the area, iii) on the basis of input from the area detector arrangement 131, the controller 130 retrieves the applicable effective time duration of the dental care action at that specific area from the data providing arrangement 120 and provides information about the effective time duration to the person. The information about the effective time duration may come in various forms, including a form of a signal indicating that the effective time duration had ended and that the person may manipulate the appliance 110 so as to move the functional unit to another area. The controller 130 may be programmed to follow algorithms aimed at providing a warning to the person if the functional unit is moved away from an area too soon or if the functional unit is kept at an area longer than necessary, to keep track of which areas have been covered and which areas still need to be addressed and to keep the person informed of the coverage, etc.

All in all, the system 100 according to the invention provides a possibility to perform a dental care procedure in such a way that a division of a total operation time over various areas in the mouth 200 that is in compliance with the extent to which oral care is needed in those various areas is realized. On the basis thereof, effectiveness of the oral care procedure can be significantly increased. Operation data can be based on data relating to a representative population and/or may be user-specific data as may be input to the system 100 by a variety of persons, particularly dental practitioners, patients themselves and home-users of the system 100. The need for dental care in a specific area may be valued on the basis of features which are related to a presence of bacteria in that area, such as a possible presence of periodontitis or a size of a possible pocket, although it is also possible to rely on other features.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The term "comprise" as used in this text will be understood by a person skilled in the art as covering the term "consist of". Hence, the term "comprise" may in respect of an embodiment mean "consist of", but may in another embodiment mean "contain/include at least the defined species and optionally one or more other species".

In the example of respective teeth being subjected to a dental care action such as brushing by means of a toothbrush, it is implicit that one area after another is subjected to the dental care action. However, this should not be understood so as to imply that the invention does not cover options of dental care actions being performed at various areas in one time so that time periods in which the dental care actions are performed overlap.

Notable aspects of the invention can be summarized as follows. In the context of dental care, a system 100 is provided which comprises i) an appliance 110 that is configured to operate a functional unit, ii) a data providing arrangement 120 that is configured to include operation data in the form of respective combinations of a mouth area and an effective time duration of a dental care action for the area concerned, wherein the effective time durations of the respective combinations add up to a total operation time of a dental care procedure and have respective values according to a proportional division of the total operation time on the basis of a distribution of the extent to which dental care is needed at the areas of the respective combinations, and iii) a controller 130 that is configured to communicate with the data providing arrangement 120 and to control operation of the appliance 110 in accordance with the operation data.

The area of the user's mouth may be a tooth, for example, and the effective time duration attributed to each tooth may be based on the respective tooth's likelihood of developing deep pockets. Alternatively or additionally, other factors which are indicative of onset or presence of gum disease can also be used to determine attribution of care time to respective mouth areas. Examples of such other factors include likelihood of plaque formation, bleeding and gum recession on a given tooth or sets thereof. Further, knowledge of a user's personal information and/or dental information can be used as input for the purpose of generating a division of the total operation time that fits the user's profile.

## Claims

1. System (100) that is configured to be operated in a context of a dental care procedure to be performed in the mouth (200) of a user for a total operation time, the dental care procedure including dental care actions to be performed at respective areas in the user's mouth by means of a functional unit, and the system (100) comprising:
an appliance (110) that is configured to operate such a functional unit,
a data providing arrangement (120) that is configured to include operation data in the form of respective combinations of an area in the user's mouth (200) and an effective time duration of a dental care action to be performed at the area concerned, wherein the effective time durations of the respective combinations add up to the total operation time and have respective values according to a proportional division of the total operation time on the basis of a distribution of the extent to which dental care is needed at the areas of the respective combinations, wherein at least two effective time durations are different from one another, and
a controller (130) that is configured to communicate with the data providing arrangement (120) and to control operation of the appliance (110) in accordance with the operation data wherein the controller (130) is configured to execute an algorithm for adjusting the operation data in the data providing arrangement (120) on the basis of input in respect of at least one area in the user's mouth (200), which algorithm comprises the steps of:
adjusting the extent to which dental care is needed in respect of the at least one areas to which the input applies,
determining a new distribution of the extent to which dental care is needed at the areas of the respective combinations, and
making a new proportional division of the total operation time on the basis of the new distribution of the extent to which dental care is needed at the areas of the respective combinations.

2. System (100) according to claim 1, wherein the distribution of the extent to which dental care is needed at the areas of the respective combinations is based on a distribution of likelihood of disease development at the areas of the respective combinations.

3. System (100) according to claim 1 or 2, wherein the distribution of the extent to which dental care is needed at the areas of the respective combinations is based on knowledge of the user's personal information and/or dental information.

4. System (100) according to any of claims 1-3, wherein the controller (130) is configured to provide the data providing arrangement (120) with input relating to an actual area where a dental care action is performed during operation and to retrieve output relating to an actual effective time duration of the dental care action from the data providing arrangement (120).

5. System (100) according to any of claims 1-4, wherein the controller (130) comprises an area detector arrangement (131) that is configured to detect an actual area where a dental care action is performed during operation.

6. System (100) according to any of claims 1-5, comprising a user interface (140) that is configured to allow a person to adjust the operation data in the data providing arrangement (120).

7. System (100) according to any of claims 1-6, comprising a communication arrangement (150) that is configured to alert a person to the expiry of an effective time duration at an actual area where a dental care action is performed during operation.

8. System (100) according to any of claims 1-7, wherein the respective areas of the respective combinations relate to respective teeth and/or respective mouth segments (A, B, C, D, E, F) including at least two teeth.

9. System (100) according to any of claims 1-8, comprising a remote device on which an app is installed that is programmed to receive input provided by a person and convey such input to the data providing arrangement (120) and/or the controller (130), and/or to provide information to a person including information about effective time durations during operation of the system (100).

10. System (100) according to any of claims 1-9, comprising the functional unit that is configured to perform the dental care actions included in the dental care procedure.

11. Controller (130) that is configured to execute an algorithm for dividing a total operation time of a dental care procedure to be performed in a person's mouth (200) in effective time durations of a dental care action per area in the person's mouth (200), which algorithm is designed to process information about a distribution of the extent to which dental care is needed over the respective areas in the person's mouth (200), and to determine operation data in the form of respective combinations of an area in the person's mouth (200) and an effective time duration of the dental care action to be performed at the area concerned by making a proportional division of the total operation time on the basis of the distribution of the extent to which dental care is needed over the respective areas in the person's mouth (200), wherein at least two effective time durations are different from one another and wherein the controller (130) is further configured to execute an algorithm for adjusting the operation data on the basis of input in respect of at least one area in the user's mouth (200), which algorithm comprises the steps of:
adjusting the extent to which dental care is needed in respect of the at least one areas to which the input applies,
determining a new distribution of the extent to which dental care is needed at the areas of the respective combinations, and
making a new proportional division of the total operation time on the basis of the new distribution of the extent to which dental care is needed at the areas of the respective combinations.

12. Remote device, comprising the controller (130) according to claim 11 and being configured to communicate with a system (100) that is configured to be operated in a context of a dental care procedure to be performed in the mouth (200) of a user for a total operation time, the dental care procedure including dental care actions to be performed at respective areas in the user's mouth by means of a functional unit, and the system (100) comprising an appliance (110) that is configured to operate such a functional unit, wherein the controller (130) is configured to control operation of the appliance (110) in accordance with the operation data.

13. Appliance (110) that is configured to operate a functional unit for the purpose of performing dental care actions at respective areas in a user's mouth, the appliance (110) comprising the controller (130) according to claim 11, wherein the controller (130) is configured to control operation of the appliance (110) in accordance with the operation data.

14. Computer program product comprising code to cause a controller (130), when the code is executed on the controller (130), to execute an algorithm for dividing a total operation time of a dental care procedure to be performed in a person's mouth (200) in effective time durations of a dental care action per area in the person's mouth (200), which algorithm is designed to process information about a distribution of the extent to which dental care is needed over the respective areas in the person's mouth (200), and to determine operation data in the form of respective combinations of an area in the person's mouth (200) and an effective time duration of the dental care action to be performed at the area concerned by making a proportional division of the total operation time on the basis of the distribution of the extent to which dental care is needed over the respective areas in the person's mouth (200), wherein at least two effective time durations are different from one another and wherein the controller (130) is further configured to execute an algorithm for adjusting the operation data on the basis of input in respect of at least one area in the user's mouth (200), which algorithm comprises the steps of:
adjusting the extent to which dental care is needed in respect of the at least one areas to which the input applies,
determining a new distribution of the extent to which dental care is needed at the areas of the respective combinations, and
making a new proportional division of the total operation time on the basis of the new distribution of the extent to which dental care is needed at the areas of the respective combinations.

## Patentansprüche

1. System (100), das dazu konfiguriert ist, in einem Kontext einer Zahnpflegeprozedur betrieben zu werden, die in dem Mund (200) eines Benutzers für eine Gesamtbetriebszeit durchzuführen ist, wobei die Zahnpflegeprozedur Zahnpflegehandlungen einschließt, die mittels einer Funktionseinheit in jeweiligen Bereichen in dem Mund des Benutzers durchzuführen sind, und das System (100) umfassend:
ein Gerät (110), das dazu konfiguriert ist, eine derartige Funktionseinheit zu betreiben,
eine Datenbereitstellungsanordnung (120), die dazu konfiguriert ist, Betriebsdaten in Form von jeweiligen Kombinationen eines Bereichs in dem Mund des Benutzers (200) und einer effektiven Zeitdauer einer in dem betreffenden Bereich durchzuführenden Zahnpflegehandlung einzuschließen, wobei die effektiven Zeitdauern der jeweiligen Kombinationen sich zu der Gesamtbetriebszeit aufaddieren und jeweilige Werte nach einer proportionalen Aufteilung der Gesamtbetriebszeit auf der Grundlage einer Verteilung des Ausmaßes aufweisen, in dem Zahnpflege in den Bereichen der jeweiligen Kombinationen erforderlich ist, wobei sich mindestens zwei effektive Zeitdauern voneinander unterscheiden, und
eine Steuereinheit (130), die dazu konfiguriert ist, mit der Datenbereitstellungsanordnung (120) zu kommunizieren und einen Betrieb des Geräts (110) nach den Betriebsdaten zu steuern, wobei die Steuereinheit (130) dazu konfiguriert ist, einen Algorithmus zum Anpassen der Betriebsdaten in der Datenbereitstellungsanordnung (120) auf der Grundlage einer Eingabe in Bezug auf mindestens einen Bereich in dem Mund (200) des Benutzers auszuführen, wobei der Algorithmus die Schritte umfasst:
Anpassen des Ausmaßes, in dem Zahnpflege in Bezug auf den mindestens einen Bereich erforderlich ist, für den die Eingabe gilt,
Bestimmen einer neuen Verteilung des Ausmaßes, in dem Zahnpflege in den Bereichen der jeweiligen Kombinationen erforderlich ist, und
Vornehmen einer neuen proportionalen Aufteilung der Gesamtbetriebszeit auf der Grundlage der neuen Verteilung des Ausmaßes, in dem Zahnpflege in den Bereichen der jeweiligen Kombinationen erforderlich ist.

2. System (100) nach Anspruch 1, wobei die Verteilung des Ausmaßes, in dem Zahnpflege in den Bereichen der jeweiligen Kombinationen erforderlich ist, auf einer Wahrscheinlichkeitsverteilung einer Krankheitsentwicklung in den Bereichen der jeweiligen Kombinationen basiert.

3. System (100) nach Anspruch 1 oder 2, wobei die Verteilung des Ausmaßes, in dem Zahnpflege in den Bereichen der jeweiligen Kombinationen erforderlich ist, auf einer Kenntnis der persönlichen Informationen und/oder Zahninformationen des Benutzers basiert.

4. System (100) nach einem der Ansprüche 1-3, wobei die Steuereinheit (130) dazu konfiguriert ist, der Datenbereitstellungsanordnung (120) eine Eingabe bezüglich eines gegenwärtigen Bereichs bereitzustellen, in dem während des Betriebs eine Zahnpflegehandlung durchgeführt wird, und eine Ausgabe bezüglich einer gegenwärtigen effektiven Zeitdauer der Zahnpflegehandlung von der Datenbereitstellungsanordnung (120) abzurufen.

5. System (100) nach einem der Ansprüche 1-4, wobei die Steuereinheit (130) eine Bereichsdetektoranordnung (131) umfasst, die dazu konfiguriert ist, einen gegenwärtigen Bereich zu detektieren, in dem während des Betriebs eine Zahnpflegehandlung durchgeführt wird.

6. System (100) nach einem der Ansprüche 1-5, das eine Benutzerschnittstelle (140) umfasst, die dazu konfiguriert ist, einer Person zu ermöglichen, die Betriebsdaten in der Datenbereitstellungsanordnung (120) anzupassen.

7. System (100) nach einem der Ansprüche 1-6, das eine Kommunikationsanordnung (150) umfasst, die dazu konfiguriert ist, eine Person auf den Ablauf einer effektiven Zeitdauer in einem gegenwärtigen Bereich aufmerksam zu machen, in dem eine Zahnpflegehandlung während des Betriebs durchgeführt wird.

8. System (100) nach einem der Ansprüche 1-7, wobei sich die jeweiligen Bereiche der jeweiligen Kombinationen auf jeweilige Zähne und/oder jeweilige Mundsegmente (A, B, C, D, E, F), die mindestens zwei Zähne einschließen, beziehen.

9. System (100) nach einem der Ansprüche 1-8, umfassend eine entfernte Vorrichtung, auf der eine App installiert ist, die dazu programmiert ist, eine Eingabe, die durch eine Person bereitgestellt wird, zu empfangen und eine derartige Eingabe an die Datenbereitstellungsanordnung (120) und/oder die Steuereinheit (130) zu übermitteln, und/oder einer Person Informationen einschließlich Informationen über effektive Zeitdauern während des Betriebs des Systems (100) bereitzustellen.

10. System (100) nach einem der Ansprüche 1-9, das die Funktionseinheit umfasst, die dazu konfiguriert ist, die in der Zahnpflegeprozedur eingeschlossene Zahnpflegehandlungen durchzuführen.

11. Steuereinheit (130), die dazu konfiguriert ist, einen Algorithmus zum Aufteilen einer Gesamtbetriebszeit einer in dem Mund (200) einer Person durchzuführenden Zahnpflegeprozedur in effektive Zeitdauern einer Zahnpflegehandlung pro Bereich in dem Mund (200) der Person auszuführen, wobei der Algorithmus dazu ausgestaltet ist, Informationen über eine Verteilung des Ausmaßes, in dem Zahnpflege über die jeweiligen Bereiche in dem Mund (200) der Person erforderlich ist, zu verarbeiten und Betriebsdaten in Form von jeweiligen Kombinationen aus einem Bereich in dem Mund (200) der Person und einer effektiven Zeitdauer der in dem betreffenden Bereich durchzuführenden Zahnpflegehandlung zu bestimmen, indem er eine proportionale Aufteilung der Gesamtbetriebszeit auf der Grundlage der Verteilung des Ausmaßes, in dem Zahnpflege über die jeweiligen Bereiche in dem Mund (200) der Person erforderlich ist, vornimmt, wobei sich mindestens zwei effektive Zeitdauern voneinander unterscheiden und wobei die Steuereinheit (130) weiter dazu konfiguriert ist, einen Algorithmus zum Anpassen der Betriebsdaten auf der Grundlage einer Eingabe in Bezug auf mindestens einen Bereich in dem Mund (200) des Benutzers auszuführen, wobei der Algorithmus die Schritte umfasst:
Anpassen des Ausmaßes, in dem Zahnpflege in Bezug auf den mindestens einen Bereich erforderlich ist, für den die Eingabe gilt,
Bestimmen einer neuen Verteilung des Ausmaßes, in dem Zahnpflege in den Bereichen der jeweiligen Kombinationen erforderlich ist, und
Vornehmen einer neuen proportionalen Aufteilung der Gesamtbetriebszeit auf der Grundlage der neuen Verteilung des Ausmaßes, in dem Zahnpflege in den Bereichen der jeweiligen Kombinationen erforderlich ist.

12. Entfernte Vorrichtung, die die Steuereinheit (130) nach Anspruch 11 umfasst und dazu konfiguriert ist, mit einem System (100) zu kommunizieren, das dazu konfiguriert ist, in einem Kontext einer in dem Mund (200) eines Benutzers für eine Gesamtbetriebszeit durchzuführenden Zahnpflegeprozedur betrieben zu werden, wobei die Zahnpflegeprozedur mittels einer Funktionseinheit an jeweiligen Bereichen in dem Mund des Benutzers durchzuführende Zahnpflegehandlungen einschließt, und das System (100) ein Gerät (110) umfasst, das dazu konfiguriert ist, eine derartige Funktionseinheit zu betreiben, wobei die Steuereinheit (130) dazu konfiguriert ist, den Betrieb des Geräts (110) nach den Betriebsdaten zu steuern.

13. Gerät (110), das dazu konfiguriert ist, eine Funktionseinheit zum Zweck eines Durchführens von Zahnpflegehandlungen in jeweiligen Bereichen in dem Mund eines Benutzers zu betreiben, wobei das Gerät (110) die Steuereinheit (130) nach Anspruch 11 umfasst, wobei die Steuereinheit (130) dazu konfiguriert ist, den Betrieb des Geräts (110) nach den Betriebsdaten zu steuern.

14. Computerprogrammprodukt, das Code umfasst, um eine Steuereinheit (130) zu veranlassen, wenn der Code auf der Steuereinheit (130) ausgeführt wird, einen Algorithmus zum Aufteilen einer Gesamtbetriebszeit einer in dem Mund einer Person (200) durchzuführenden Zahnpflegeprozedur in effektive Zeitdauern einer Zahnpflegehandlung pro Bereich in dem Mund der Person (200) auszuführen, wobei der Algorithmus dazu ausgestaltet ist, Informationen über eine Verteilung des Ausmaßes, in dem Zahnpflege über die jeweiligen Bereiche in dem Mund (200) der Person erforderlich ist, zu verarbeiten und Betriebsdaten in Form von jeweiligen Kombinationen aus einem Bereich in dem Mund (200) der Person und einer effektiven Zeitdauer der in dem betreffenden Bereich durchzuführenden Zahnpflegehandlung zu bestimmen, indem er eine proportionale Aufteilung der Gesamtbetriebszeit auf der Grundlage der Verteilung des Ausmaßes, in dem Zahnpflege über die jeweiligen Bereiche in dem Mund (200) der Person erforderlich ist, vornimmt, wobei sich mindestens zwei effektive Zeitdauern voneinander unterscheiden und wobei die Steuereinheit (130) weiter dazu konfiguriert ist, einen Algorithmus zum Anpassen der Betriebsdaten auf der Grundlage einer Eingabe in Bezug auf mindestens einen Bereich in dem Mund (200) des Benutzers auszuführen, wobei der Algorithmus die Schritte umfasst:
Anpassen des Ausmaßes, in dem Zahnpflege in Bezug auf den mindestens einen Bereich erforderlich ist, für den die Eingabe gilt,
Bestimmen einer neuen Verteilung des Ausmaßes, in dem Zahnpflege in den Bereichen der jeweiligen Kombinationen erforderlich ist, und
Vornehmen einer neuen proportionalen Aufteilung der Gesamtbetriebszeit auf der Grundlage der neuen Verteilung des Ausmaßes, in dem Zahnpflege in den Bereichen der jeweiligen Kombinationen erforderlich ist.

## Revendications

1. Système (100) qui est configuré pour fonctionner dans le cadre d'une opération de soins dentaires à réaliser dans la bouche (200) d'un utilisateur pendant un temps de fonctionnement total, l'opération de soins dentaires incluant des actions de soins dentaires à réaliser dans des zones respectives de la bouche de l'utilisateur au moyen d'une unité fonctionnelle, et le système (100) comprenant :
un appareil (110) qui est configuré pour faire fonctionner une telle unité fonctionnelle,
un ensemble de fourniture de données (120) qui est configuré pour inclure des données de fonctionnement sous la forme de combinaisons respectives d'une zone dans la bouche (200) de l'utilisateur et d'une durée effective d'une action de soins dentaires à effectuer dans la zone concernée, dans lequel les durées effectives des combinaisons respectives s'additionnent pour former le temps de fonctionnement total et présentent des valeurs respectives selon une division proportionnelle du temps de fonctionnement total sur la base d'une distribution de la mesure dans laquelle les soins dentaires sont nécessaires dans les zones des combinaisons respectives, dans lequel au moins deux durées effectives sont différentes l'une de l'autre, et
un dispositif de commande (130) qui est configuré pour communiquer avec l'ensemble de fourniture de données (120) et pour commander le fonctionnement de l'appareil (110) en fonction des données de fonctionnement, dans lequel le dispositif de commande (130) est configuré pour exécuter un algorithme permettant d'ajuster les données de fonctionnement de l'ensemble de fourniture de données (120) sur la base d'une entrée concernant au moins une zone de la bouche (200) de l'utilisateur, lequel algorithme comprend les étapes consistant à :
ajuster la mesure dans laquelle les soins dentaires sont nécessaires en ce qui concerne la au moins une zone à laquelle l'entrée s'applique,
déterminer une nouvelle distribution de la mesure dans laquelle les soins dentaires sont nécessaires dans les zones des combinaisons respectives, et
effectuer une nouvelle division proportionnelle du temps de fonctionnement total sur la base de la nouvelle distribution de la mesure dans laquelle les soins dentaires sont nécessaires dans les zones des combinaisons respectives.

2. Système (100) selon la revendication 1, dans lequel la distribution de la mesure dans laquelle les soins dentaires sont nécessaires dans les zones des combinaisons respectives est basée sur une distribution de probabilité de développement de maladie dans les zones des combinaisons respectives.

3. Système (100) selon la revendication 1 ou 2, dans lequel la distribution de la mesure dans laquelle les soins dentaires sont nécessaires dans les zones des combinaisons respectives est basée sur la connaissance des informations personnelles et/ou des informations dentaires de l'utilisateur.

4. Système (100) selon l'une quelconque des revendications 1-3, dans lequel le dispositif de commande (130) est configuré pour fournir à l'ensemble de fourniture de données (120) une entrée relative à une zone réelle où une action de soins dentaires est effectuée pendant le fonctionnement et pour récupérer une sortie relative à une durée effective réelle de l'action de soins dentaires à partir de l'ensemble de fourniture de données (120).

5. Système (100) selon l'une quelconque des revendications 1-4, dans lequel le dispositif de commande (130) comprend un ensemble de détecteur de zone (131) qui est configuré pour détecter une zone réelle où une action de soins dentaires est effectuée pendant le fonctionnement.

6. Système (100) selon l'une quelconque des revendications 1-5, comprenant une interface utilisateur (140) qui est configurée pour permettre à une personne d'ajuster les données de fonctionnement dans l'ensemble de fourniture de données (120).

7. Système (100) selon l'une quelconque des revendications 1-6, comprenant un ensemble de communication (150) qui est configuré pour alerter une personne de l'expiration d'une durée effective dans une zone réelle où une action de soins dentaires est effectuée pendant le fonctionnement.

8. Système (100) selon l'une quelconque des revendications 1-7, dans lequel les zones respectives des combinaisons respectives se rapportent à des segments de dents respectifs et/ou de bouche respectifs (A, B, C, D, E, F) incluant au moins deux dents.

9. Système (100) selon l'une quelconque des revendications 1-8, comprenant un dispositif distant sur lequel est installée une application qui est programmée pour recevoir une entrée fournie par une personne et transmettre une telle entrée à l'ensemble de fourniture de données (120) et/ou au dispositif de commande (130), et/ou pour fournir des informations à une personne, incluant des informations sur les durées effectives pendant le fonctionnement du système (100).

10. Système (100) selon l'une quelconque des revendications 1-9, comprenant l'unité fonctionnelle qui est configurée pour effectuer les actions de soins dentaires incluses dans l'opération de soins dentaires.

11. Dispositif de commande (130) qui est configuré pour exécuter un algorithme de division d'un temps de fonctionnement total d'une opération de soins dentaires à réaliser dans la bouche (200) d'une personne en durées effectives d'une action de soins dentaires par zone de la bouche (200) de la personne, lequel algorithme est conçu pour traiter des informations sur une distribution de la mesure dans laquelle les soins dentaires sont nécessaires sur les zones respectives de la bouche (200) de la personne, et pour déterminer des données de fonctionnement sous la forme de combinaisons respectives d'une zone de la bouche (200) de la personne et d'une durée effective de l'action de soins dentaires à réaliser sur la zone concernée en effectuant une division proportionnelle du temps de fonctionnement total sur la base de la distribution de la mesure dans laquelle les soins dentaires sont nécessaires sur les zones respectives de la bouche (200) de la personne, dans lequel au moins deux durées effectives sont différentes l'une de l'autre et dans lequel le dispositif de commande (130) est en outre configuré pour exécuter un algorithme d'ajustement des données de fonctionnement sur la base de l'entrée relative à au moins une zone de la bouche (200) de l'utilisation, lequel algorithme comprend les étapes consistant à :
ajuster la mesure dans laquelle les soins dentaires sont nécessaires en ce qui concerne la au moins une zone à laquelle l'entrée s'applique,
déterminer une nouvelle distribution de la mesure dans laquelle les soins dentaires sont nécessaires dans les zones des combinaisons respectives, et
effectuer une nouvelle division proportionnelle du temps de fonctionnement total sur la base de la nouvelle distribution de la mesure dans laquelle les soins dentaires sont nécessaires dans les zones des combinaisons respectives.

12. Dispositif à distance, comprenant le dispositif de commande (130) selon la revendication 11 et étant configuré pour communiquer avec un système (100) qui est configuré pour être utilisé dans un contexte d'une opération de soins dentaires à effectuer dans la bouche (200) d'un utilisateur pendant un temps de fonctionnement total, l'opération de soins dentaires incluant des actions de soins dentaires à effectuer dans des zones respectives de la bouche de l'utilisateur au moyen d'une unité fonctionnelle, et le système (100) comprenant un appareil (110) qui est configuré pour faire fonctionner une telle unité fonctionnelle, dans lequel le dispositif de commande (130) est configuré pour commander le fonctionnement de l'appareil (110) conformément aux données de fonctionnement.

13. Appareil (110) qui est configuré pour faire fonctionner une unité fonctionnelle dans le but d'effectuer des actions de soins dentaires dans des zones respectives de la bouche d'un utilisateur, l'appareil (110) comprenant le dispositif de commande (130) selon la revendication 11, dans lequel le dispositif de commande (130) est configuré pour commander le fonctionnement de l'appareil (110) conformément aux données de fonctionnement.

14. Produit de programme informatique comprenant un code permettant d'amener un dispositif de commande (130), lorsque le code est exécuté sur le dispositif de commande (130), à exécuter un algorithme de division d'un temps de fonctionnement total d'une opération de soins dentaires à réaliser dans la bouche (200) d'une personne en durées effectives d'une action de soins dentaires par zone de la bouche (200) de la personne, lequel algorithme est conçu pour traiter des informations sur une distribution de la mesure dans laquelle les soins dentaires sont nécessaires sur les zones respectives de la bouche (200) de la personne, et pour déterminer des données de fonctionnement sous la forme de combinaisons respectives d'une zone de la bouche (200) de la personne et d'une durée effective de l'action de soins dentaires à réaliser sur la zone concernée en effectuant une division proportionnelle du temps de fonctionnement total sur la base de la distribution de la mesure dans laquelle les soins dentaires sont nécessaires sur les zones respectives de la bouche (200) de la personne, dans lequel au moins deux durées effectives sont différentes l'une de l'autre et dans lequel le dispositif de commande (130) est en outre configuré pour exécuter un algorithme d'ajustement des données de fonctionnement sur la base de l'entrée relative à au moins une zone de la bouche (200) de l'utilisation, lequel algorithme comprend les étapes consistant à :
ajuster la mesure dans laquelle les soins dentaires sont nécessaires en ce qui concerne la au moins une zone à laquelle l'entrée s'applique,
déterminer une nouvelle distribution de la mesure dans laquelle les soins dentaires sont nécessaires dans les zones des combinaisons respectives, et
effectuer une nouvelle division proportionnelle du temps de fonctionnement total sur la base de la nouvelle distribution de la mesure dans laquelle les soins dentaires sont nécessaires dans les zones des combinaisons respectives.
